# EUROPEAN PATENT SPECIFICATION

(11) **EP 0 986 565 B1**
(45) Date of publication and mention of the grant of the patent: **14.01.2009**
(21) Application number: 98932105.4
(22) Date of filing: 02.06.1998
(51) Int. Cl.: C07D 501/00

(54) **IMPROVED PRECIPITATION PROCESS OF 7-AMINOCEPHALOSPORANIC ACID (7-ACA)**
VERBESSERTES FÄLLUNGSVERFAHREN VON 7-AMINOCEPHALOSPORANSÄURE
PROCEDE DE PRECIPITATION AMELIORE D'UN ACIDE 7-AMINOCEPHALOSPORANIQUE (7-ACA)

(30) Priority: 04.06.1997 AT 95197; 30.06.1997 AT 111297
(43) Date of publication of application: 22.03.2000
(73) Proprietor: SANDOZ AG, 4002 Basel (CH)
(72) Inventor: NADENIK, Petr, A-6300 Wörgl (AT); WAGNER, Helmut, A-6233 Kramsach (AT)
(86) International application number: PCT/EP1998/003278
(87) International publication number: WO 1998/055484

(56) References cited:
- DE-A- 1 545 898
- DE-A- 2 222 094
- DE-A- 2 502 097
- DE-A- 3 002 659
- GB-A- 1 277 228
- GB-A- 1 421 199
- CHEMICAL ABSTRACTS, vol. 90, no. 15, 9 April 1979 Columbus, Ohio, US; abstract no. 121587p, CIESLAK J ET AL : "Isolation of beta-lactam antibiotics from aqueous solutions." page 646; column 2; XP002079393 & PL 93 437 A (INSTYTUT PRZEMYSLO FARAMCEUTYCZNEGO) 30 November 1977
- DATABASE WPI Section Ch, Week 9312 Derwent Publications Ltd., London, GB; Class B02, AN 93-098389 XP002079394 & RO 102 148 A (INST CERC ANTIBIOTICE IASI), 30 September 1991
- OBERHAUSER T ET AL: "On the Stereochemical Purity of (+)-7-Aminocephalosporanic Acid" TETRAHEDRON, vol. 52, no. 22, 27 May 1996, page 7691-7702 XP004106223
- DATABASE WPI Section Ch, Week 9424 Derwent Publications Ltd., London, GB; Class B02, AN 94-200170 XP002079395 & WO 94 12504 A (FUJISAWA PHARM CO LTD) , 9 June 1994 & WO 94 12501 A (FUJISAWA PHARMACEUTICAL CO LTD) 9 June 1994

## Description

The present invention relates to 7-aminocephalosporanic acid (7-ACA) 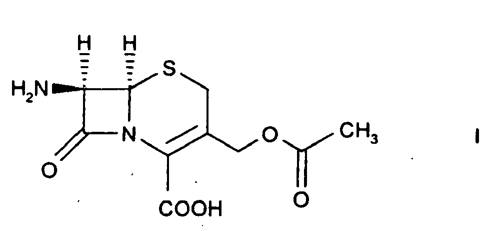 7-ACA is a key intermediate compound in the synthesis of many semi-synthetic cephalosporin antibiotics. It may e.g. be produced from cephalosporin C by cleavage of the amide function in position 7 of the ring sytem
- e.g. chemically, e.g. by conversion of the amide function into an imide chloride function which may be hydrolysed to give 7-ACA, e.g. in a strong acidic medium and precipitating 7-ACA, e.g. by adjustment of the pH (around the isoelectric point), e.g. by addition of a base; 7-ACA may be isolated in the form of rosettes and agglomerates;
- or enzymatically, e.g. by action of an acylase; or by conversion of cephalosporin C into glutaryl-7-amino-cephalosporanic acid, e.g. by action of a D-amino acid oxidase, enzymatically hydrolysing a glutaryl-7-amino cephalosporanic acid to obtain 7-ACA, e.g. in a basic medium, neutral or slightly acidic medium, optionally purifying the reaction solution with an appropriate ion exchanger or adsorber resin, and precipitating 7-ACA, e.g. by adjustment of the pH (around the isoelectric point), e.g. by addition of an acid, e.g. HCl. In that case precipitated 7-ACA may be, however, in the form of very small, loose, needle-like crystals difficult to be isolated. Increasing purity of 7-ACA may result in still smaller crystals and addition of an organic solvent, e.g. a, e.g. lower, alcohol, e.g. methanol or a ketone, e.g. acetone, to the reaction mixture before the isolation of 7-ACA, which may improve the yield, again may result in considerably smaller crystals.

Chem. Abstr. 90:121587 (1979) discloses a process for isolation of penicillins or cephalosporins by subsequent extractions of their aqueous solutions, first with the guanidinium compound p-[R³NHCH(CCl₃)]C₆H₄CH:NNHC(:NH)NH₂ CH₃COOH and secondly with a sodium- or potassium-salt of tri-haloacetic acid, e.g. CCl₃COOK, followed by a pH adjustment. The corresponding tri-haloacetic acid may be formed under acidic conditions.

GB 1277228 describes a process for cleavage of cephalosporin C with a nitrosating agent, followed by addition of a lower alkanol and precipitation of 7-ACA from the alcoholic solution. Specifically in examples 1-6 the process involves cleavage of the cephalosporin C in a formic acid solution. 2,2-dimethoxypropane or methylformate is added and the pH is adjusted to pH 3.5 with ammonium hydroxide to precipitate 7-ACA.

RO102148 describes a process for the purification of 7-ACA by dissolving 7-ACA in a halogenated aliphatic lower alkyl hydrocarbon solvent in the presence of e.g. triethylamine followed by an activated charcoal treatment and precipitation of the tri-ethylamin-salt of 7-ACA by adding a hydrocarbon as countersolvent. The obtained tri-ethylamin-salt is dissolved in water and the pH is decreased to be near the isoelectric point of 7-ACA.

DE3002659 concerns a process for the production of 7-ACA starting from Cephalosporin C or ester derivatives thereof. In example 11 of DE3002659, triethylamine is added as a base to adjust the acidic solution to pH 3.3. Prior to the addition of triethylamine the pH was described to be pH 2.0 due to the formic acid and triethylamine.

DE2222094 relates to a process for the production of 7-ACA out of cephalosporin compounds acylated at the 7-amino group by protecting the 4-carboxylic group with phosphinic acid, forming a 7-imidohalide, converting it into an 7-iminoether by adding an alcohol and subsequently hydrolyzing the iminoether with water into 7-ACA. Further on, DE222094 discloses a process variant wherein during a second crystallization a bicarbonate salt is added to adjust the pH to 3.5.

Subject of GB1421199 is an improvement of processes for the production of 7-ACA from Cephalosporin C which use the reaction sequence of first protecting free carboxylic groups then cleaving the 7-(5'-aminoadipoylamido)-group with a halogenating agent to obtain an iminohalide which is further converted into an iminoether and hydrolyzed to obtain 7-ACA. The proposed improvement described in GB1421199 is to add catalytic amounts of a certain type of base such as quinoline, isoquinoline, pyridine or a picoline, prior to the addition of a halogentating reactant. Examples 2 to 4 and 6-7 of GB1421199 disclose that ethylene glycol is added before the pH value is adjusted slowly to pH 3.5 with concentrated ammonia.

DE 1545898 discloses a process for the purification of 7-ACA which comprises forming a solution of an acid addition salt of 7-ACA or of an acid addition salt from a base salt of 7-ACA with a hydrocarbon-sulphonic acid or nitric acid, removing any insoluble impurities from said solution and regenerating 7-ACA from the acid addition salt. Two alternative ways for recovering the 7-ACA are disclosed: 1. dissolving the acid addition salt of 7-ACA in a lower alkanol and adjusting the pH of the solution by means of a base, e.g. aqueous ammonia, to about pH3.5, or 2. dissolving the acid addition salt of 7-ACA in N,N-dimethylacetamide or N,N.dimethylformamide and pouring the obtained solution into water.
Tetrahedron , Vol 52 (No. 22) (1996), pp. 7691-7702 discloses a route of chemical synthesis for the preparation of 7-ACA. In order to isolate 7-ACA, it is described to dissolve a potassium salt of 7-ACA in ice-cold water and then to decrease the pH value from pH7.4 to pH 3.5 by adding of a 1 N HCl solution.

DE2502097 discloses a process for the purification of cefazolin, which is 7-[1-(1H)tetrazolylacetamido]3-[(5-methyl-1,3,4-thiadiazol-2-yl)thiomethyl]-3-cephem-4-carboxylic acid, and other 7-acyl-cephalosporins by bringing a solution of the cephalosporin to be purified into contact with a non-ionic polymeric resin adsorbent being selected from a cross linked styrene-divinylbenzene co-polymer and a cross-linked acrylic ester polymer. The pH value of the solution of said cephalosporin is adjusted if necessary to be in the range of pH 3 to pH 6.5 so that at least 99% of the cephalosporin is ionized.

The present invention provides a process, e.g. which may by carried out on technical scale, wherein agglomerates or rosettes of 7-ACA may be formed on 7-ACA precipitation from an alkaline, neutral or slightly acidic medium with a pH above the isoelectric point of 7-ACA, which substantially improves isolation of 7-ACA, e.g. by filtration and centrifugation and additionally, 7-ACA obtained according to the present invention may be dried more quickly, e.g. which may result in a smaller amount of by-products compared with 7-ACA obtained according to a prior art process.

In one aspect the present invention provides a process for the production of rosettes or agglomerates of 7-ACA, e.g. of formula I, characterized in, that 7-ACA is precipitated from slightly acidic, neutral or alkaline solution with a pH above the isoelectric point of 7-ACA in the presence of an additive, e.g. selected from a group consisting of organic carboxylic acid esters, polymeric glycols and amino acids and esters thereof e.g. groups as defined below.

An additive according to the present invention may be a compound which on addition in a precipitation process of 7-ACA may cause formation of agglomerates and/or rosettes includes e.g. organic carboxylic acid esters,e.g. of formula

R₁-COO-R₂ II,

polymeric glycols, e.g. polyethylene and polypropylene glycols, e.g of formula

H(-OCHR₁-CHR₂)ₖ -OH III,

and amino acids and esters thereof, e.g. of formula

R₁₀-CH-(NR₂R₄)-COOR₁ VIII

e.g. including mixtures of individual additives, *e.g.* as described above.

An additive may be preferably an amino acid and esters therof, e.g. an amino acid such as e.g. lysine.

### In formulae II, III and VIII

R₁, R₂, R₄ and R₅ independently of each other denote hydrogen, (C₁₋₄)alkyl, unsubstituted phenyl or phenyl substituted by hydroxy or (C₁₋₄)alkyl;
k denotes a whole number from 2 to 200;
X denotes -O- or -NR₁-;
and R₁₀ denotes hydrogen, alkyl, aryl or a group of formula -(CH₂)ₘ-X-R₅.

If not otherwise defined herein, alkyl includes e.g. (C₁₋₂₂)alkyl, such as (C₁₋₈)alkyl, e.g. lower alkyl, such as (C₁₋₄)alkyl and aryl includes e.g. phenyl, naphthyl, such as phenyl.

Alkyl and aryl includes unsubstituted alkyl and aryl and alkyl and aryl substituted by groups which do not cause the formation of another compound than 7-ACA under precipitation conditions of 7-ACA in alkaline, neutral and slightly acidic medium; e.g. which do not chemically react with 7-ACA to form another compound. Preferably alkyl includes lower alkyl; aryl includes phenyl and substituted aryl includes substituted aryl, e.g. phenyl by hydroxy or alkyl e.g. lower alkyl. Amino includes unsubstituted amino or ammonium and substituted amino and ammonium, e.g. by alkyl.

In another aspect the present invention provides a process for the isolation of 7-ACA, e.g. of formula I from slightly acidic, neutral or alkaline solution with a pH above the isoelectric point of 7-ACA, characterized in that 7-ACA is precipitated in the presence of an additive, e.g. selected from a group comprising groups as e.g. defined above, e.g. in an amount of 1 ppm to 10% in the slightly acidic, neutral or alkaline solution.

A process of the present invention may be carried out as follows:
7-ACA may be precipitated
from slightly acidic, neutral or alkaline solution of 7-ACA in a solvent having a pH which is above the isoelectric point of 7-ACA, including e.g. a solution of 7-ACA in a solvent to which an acid is to be added for precipitating 7-ACA therefrom ; which is in contrast to a strong acidic solution of 7-ACA to which a base is to be added for precipitating 7-ACA therefrom, e.g. a solution of 7-ACA having a pH which is below the isoelectric point of 7-ACA in a solvent in the presence of an additive, e.g. selected from a group comprising groups as defined above, e.g. in the presence of seed crystals of 7-ACA, e.g. in the form of rosettes and/or agglomerates by addition of an acid.
An additive according to the present invention is known or may be produced analogously to known, e.g. conventional processes. A slightly acidic, neutral or alkaline solution of 7-ACA having a pH which is above the isoelectric point of 7-ACA may be obtained e.g. by an enzymatic process as defined above. The concentration of 7-ACA in slightly acidic, neutral or alkaline solution is not critical and may vary within a broad range, including e.g. a range of, e.g. ca., 5 to 60 g/l, such as 10 to 50 g/l. An additive may be e.g. added to 7-ACA in slightly acidic, neutral or alkaline solution before addition of an acid or simultanously.

The amount of an additive according to the present invention is not critical, e.g. for ecological reasons a low amount of an additive, e.g. ca., 1 ppm to 10% (v/v) in respect with the amount of 7-ACA solution may be appropriate. In case of use of non-polymeric additives, such as e.g. organic esters as an additive an amount of, e.g. ca., 1 % to 10% may be appropriate; in case of use of an amino acid or ester thereof an amount of, e.g. ca., 0.01% to 10%, such as, e.g. ca., 0.05% to 5% may be appropriate, in case of use of a polymeric additives such as polymeric glycols e.g. ca. 1 to 100 ppm may be appropriate. Seed crystals of 7-ACA, e.g. as defined above, may be added to a solution or to a resulting crystal suspension of 7-ACA either prior to or simultaneously with an acid.

The process of the present invention may be carried out batchwise or continuously, in a broad temperature range, including e.g. -15° to 40°C, such as 0° to 25°C.

An appropriate acid includes inorganic acids, e.g. sulphuric acid, hydrochloric acid or phosphoric acid, or organic acids, e.g. acetic acid.

The acid is added in an amount which is sufficient that 7-ACA, e.g. in high yields, is precipitated from the solution. A pH of the reaction mixture of around the isoelectric point of 7-ACA in a solvent may be convenient, including, but not limited to, a pH of 3.5 to 6, such as 3.5 to 5.5. 7-ACA may precipitate on acid addition, e.g. in crystalline form. A crystal suspension obtained may be stirred, e.g. under adjustment of the pH around the isoelectric point in order to complete precipitation, e.g. under cooling.

7-ACA obtained, e.g. in crystalline form and in the form of agglomerates and/or rosettes may precipitate and may be isolated, e.g. as conventional, such as by filtration, centrifugation, washed as appropriate and dried. The drying temperatures may be low, e.g. 40° to 50°, e.g. under vacuum, and the drying times may be short, e.g. ca. 5 to 30, such as 10 to 20 hours.

The presence of an additive according to the present invention in the precipitaion of 7-ACA may surprisingly result in the formation of agglomerates or rosettes of 7-ACA, even in case that an organic solvent such as an alcohol or a ketone is present in the solution and even in case that highly pure 7-ACA, e.g. purified via an adsorber resin purification, e.g. with Amberlite XAD 1600^{R}, XE-714^{R}, Dianion HP21^{R}, Sepabeads SP825^{R}, SP850^{R}), or via an ion exchanger purification, e.g. with IRA 420^{R}. The filtration time of a 7-ACA crystal suspension in the presence of an additive obtained according to the present invention may considerably be reduced in comparison with the filtration time of 7-ACA obtained without the presence of an additive, e.g. from ca. 15 minutes to 1 minute and even below, such as of ca. 0.4 minutes.

The following examples are intended to illustrate the invention. Temperatures are given in degree Celsius and are uncorrected.

7-ACA is 7-aminocephalosporanic acid, e.g. of formula I.

### Examples 1 to 14 - General procedure

142 ml of water and 2.2 g of 7-ACA in form of rosettes and/or agglomerates as a seeding material are placed in a precipitation reactor, and the pH is adjusted to 5.5 with 1 N NaOH. A solution of 25 g of 7-ACA in the form of a sodium salt, 708 ml of water with or without methanol (in ml) as set out in TABLE 1 below having a pH of ca. 7.0 is mixed with an additive as set out in TABLE 1 below and the mixture obtained is added dropwise to the mixture in the reactor under stirring.over the course of ca. 25 minutes at ca. 18°. During addition of the 7-ACA mixture into the mixture in the precipitation reactor the pH of the reaction mixture obtained is adjusted to 5.5 by addition of 20% sulphuric acid. The pH of a suspension obtained is adjusted to 4.0, the suspension is cooled to 0° and stirred for ca. 1 hour. Crystalline 7-ACA obtained is filtrated off, washed with 120 ml of water, 120 ml of 70% methanol and 120 ml of methanol and dried in a vacuum (ca. 10 mbar) for ca. 16 hours at ca. 50°. The filtration times (in minutes) are summarised in TABLE 1 below.

**TABLE 1**

| **Example** | **Methanol [ml]** | **Additive** | **Filtration time [min]** |
|---|---|---|---|
| 1 | 212 | No additive | 15 |
| | | | |
| | | | |
| 4 | 0 | Organic carboxylic acid ester | 2.1 |
| | | e.g. 49.5 ml Ethyl acetate | |
| 5 | 0 | Organic carboxylic acid ester | 1.1 |
| | | e.g. 10.6 ml Butyl acetate | |
| 6 | 212 | Organic carboxylic acid ester | 2.4 |
| | | e.g. 13.8 ml Butyl acetate | |
| 7 | 212 | Organic carboxylic acid ester | 2.9 |
| | | e.g. 64.4 ml Ethyl acetate | |
| | | | |
| | | | |
| | | | |
| 11 | 177 | Polymeric glycol | 2.1 |
| | | e.g. 3.2 ml 1 % solution of PEG 300^{R} (Fluka) | |
| | | | |
| | | | |
| 14 | 177 | Amino acid | 3.3 |
| | | e.g. 0.8 ml 1 % solution of 1-lysine | |

### Examples 15 to 18 - General Procedure

A solution of 20.8 g of 7-ACA in the form of a sodium salt, 500 ml of water with or without methanol (in ml) as set out in TABLE 2 below having a pH of ca. 7.5 is placed in a precipitation reactor at room temperature, and mixed with an additive as set out in TABLE 2 below. The pH of the mixture obtained is adjusted to 5.5 by addition of 20% sulphuric acid under stirring and kept for ca. 20 minutes. The pH of a suspension obtained is adjusted to 3.8, the suspension is cooled to 0° and stirred for ca. 1 hour. Crystalline 7-ACA obtained is filtrated off, washed with 100 ml of water, 100 ml of 70% methanol and 100 ml of methanol and dried in a vacuum (ca. 10 mbar) for ca. 16 hours at ca. 50°. The filtration times (in minutes) are summarised in TABLE 2 below.

**TABLE 2**

| **Example** | **Methanol [ml]** | **Additive** | **Filtration time [min]** |
|---|---|---|---|
| 15 | 150 | No additive | 14 |
| 16 | 100 | Organic carboxylic acid ester | 1.5 |
| | | e.g. 9 ml 1 Butyl acetate | |
| | | | |
| | | | |

## Claims

1. A process for the isolation of 7-ACA of formula 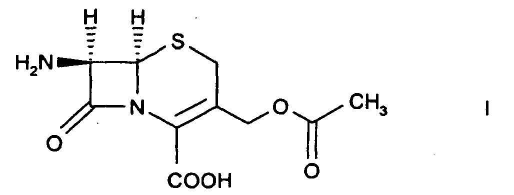 from slightly acidic, neutral or alkaline solution with a pH above the isoelectric point of 7-ACA, **characterized in that** 7-ACA is precipitated in the presence of an additive selected from the group consisting of organic carboxylic acid esters, polymeric glycols and amino acids and esters thereof.

2. A process according to claim 1 wherein 7-ACA is produced in the form of rosettes or agglomerates.

3. A process according to claims 1 or 2 **characterized in that** an additive is an amino acid.

4. A process according to claim 3 wherein the additive is lysine.

5. A process according to any one of claims 1 to 4, **characterized in that** the additive is present in an amount of 1 ppm to 10% in the alkaline, neutral or slightly acidic medium containing 7-ACA.

6. A process according to claim 1 **characterised in that** the additive is selected from
a) organic esters of the general formula
R₁-COO-R₂ II
wherein R₁ and R₂ are the same or different and represent hydrogen, (C₁₋₄)alkyl, phenyl, hydroxyphenyl or (C₁₋₄)alkylphenyl;
b) polymeric glycol compounds, preferably polyethylene and polypropylene glycol derivatives, of the general formula
H(-OCHR₁-CHR₂)ₖ -OH III
wherein R₁ and R₂ are as defined above, and k denotes a whole number from 2 to 200; or
c) amino acids and esters thereof, of the general formula
R₁₀-CH-(NR₂R₄)-COOR₁ VIII
wherein R₄ denotes hydrogen, (C₁₋₄)alkyl, unsubstituted phenyl or phenyl substituted by hydroxy or by (C₁₋₄)alkyl, R₁₀ denotes hydrogen, alkyl, aryl or a group of formula -(CH₂)ₘ-X-R₅ wherein X denotes -O- or -NR₁-, and R₅ denotes hydrogen, (C₁₋₄)alkyl, unsubstituted phenyl or phenyl substituted by hydroxy or by (C₁₋₄)alkyl, and the remaining substituents are as defined above, and the additive is added during precipitation.

## Patentansprüche

1. Verfahren zur Isolierung von 7-ACA der Formel 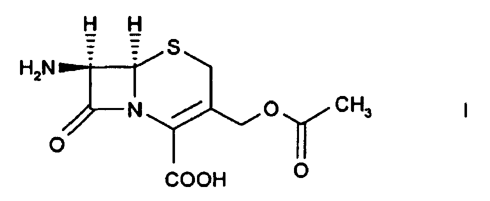 aus leicht saurer, neutraler oder alkalischer Lösung mit einem pH-Wert oberhalb des isoelektrischen Punkts von 7-ACA, **dadurch gekennzeichnet, dass** 7-ACA ausgefällt wird in Gegenwart eines Additivs, das ausgewählt ist aus der Gruppe, die besteht aus organischen Carbonsäureestern, polymeren Glycolen und Aminosäuren und Estern hiervon.

2. Verfahren nach Anspruch 1, worin 7-ACA in Form von Rosetten oder Agglomeraten hergestellt wird.

3. Verfahren nach Anspruch 1 oder 2, **dadurch gekennzeichnet, dass** ein Additiv eine Aminosäure ist.

4. Verfahren nach Anspruch 3, worin das Additiv Lysin ist.

5. Verfahren nach einem der Ansprüche 1 bis 4, **dadurch gekennzeichnet, dass** das Additiv vorhanden ist in einer Menge von 1 ppm bis 10 % in dem alkalischen, neutralen oder leicht sauren Medium, das 7-ACA enthält.

6. Verfahren nach Anspruch 1, **dadurch gekennzeichnet, dass** das Additiv ausgewählt ist aus
a) organischen Estern der allgemeinen Formel
R₁-COO-R₂ II
worin R₁ und R₂ gleich oder verschieden sind und für Wasserstoff, (C₁-C₄)-Alkyl, Phenyl, Hydroxyphenyl oder (C₁-C₄)-Alkylphenyl stehen,
b) polymeren Glycolverbindungen, vorzugsweise Polyethylenglycolderivaten und Polypropylenglycolderivaten, der allgemeinen Formel
H(-OCHR₁-CHR₂)ₖ-OH III
worin R₁ und R₂ wie oben definiert sind und k für eine ganze Zahl von 2 bis 200 steht, oder
c) Aminosäuren und Estern hiervon der allgemeinen Formel
R₁₀-CH-(NR₂R₄)-COOR₁ VIII
worin R₄ für Wasserstoff, (C₁-C₄)-Alkyl, unsubstituiertes Phenyl oder Phenyl steht, das substituiert ist mit Hydroxy oder mit (C₁-C₄)-Alkyl, R₁₀ für Wasserstoff, Alkyl, Aryl oder eine Gruppe der Formel -(CH₂)ₘ-X-R₅ steht, worin X für -O- oder -NR₁- steht und R₅ für Wasserstoff, (C₁-C₄)-Alkyl, unsubstituiertes Phenyl oder Phenyl steht, das mit Hydroxy oder mit (C₁-C₄)-Alkyl substituiert ist, und die restlichen Substituenten wie oben definiert sind, und das Additiv während der Ausfällung zugegeben wird.

## Revendications

1. Un procédé pour l'isolation de 7-ACA de formule 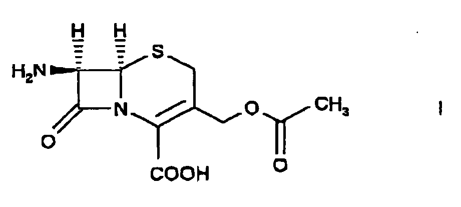 à partir d'une solution légèrement acide, neutre ou alcaline avec un pH supérieur au point isoélectrique de 7-ACA, **caractérisé en ce que** le 7-ACA est précipité en présence d'un additif sélectionné du groupe formé par des esters d'acides carboxyliques organiques, des glycols polymériques et des acides aminés et des esters de ceux-ci.

2. Un procédé selon la revendication 1, dans lequel le 7-ACA est produit sous forme de rosettes ou d'agglomérats.

3. Un procédé selon les revendications 1 ou 2, **caractérisé en ce qu'**un additif est un acide aminé.

4. Un procédé selon la revendication 3, dans lequel l'additif est la lysine.

5. Un procédé selon l'une quelconque des revendications 1 à 4, **caractérisé en ce que** l'additif est présent en une quantité de 1 ppm à 10 % dans le milieu alcalin, neutre ou légèrement acide contenant le 7-ACA.

6. Un procédé selon revendication 1, **caractérisé en ce que** l'additif est sélectionné à partir :
a. d'esters organiques de formule générale
**R₁-COO-R₂** **II**
dans laquelle R₁ et R₂ sont les mêmes ou différents et représentent hydrogène, (C₁₋₄) alkyle, phényle, hydroxyphényle ou (C₁₋₄) alkylphényle ;
b. des composés glycols polymériques, de préférence des dérivés de polyéthylène et polypropylène glycol de formule générale
**H(-OCHR₁-CHR₂)ₖ-OH** **III**
dans laquelle R₁ et R₂ sont comme définis précédemment et k représente un nombre entier de 2 à 200 ; ou
c. des acides aminés et des esters de ceux-ci de formule générale
**R₁₀-CH-(NR₂R₄)-COOR₁** **VIII**
dans laquelle R₄ représente hydrogène, (C₁₋₄) alkyle, phényle non substitué ou phényle substitué par hydroxy ou par (C₁₋₄)alkyle, R₁₀ représente hydrogène, alkyle, aryle ou un groupe de formule -(CH₂)ₘ-X-R₅ dans laquelle X représente -O- ou -NR₁- et R₅ représente hydrogène, (C₁₋₄) alkyle, phényle non substitué ou phényle substitué par hydroxy ou par (C₁₋₄)alkyle et les substituants restants sont comme définis précédemment et l'additif est ajouté durant la précipitation.
